# EUROPÄISCHE PATENTANMELDUNG

(11) **EP 3 498 752 A1**
(43) Veröffentlichungstag der Anmeldung: **19.06.2019**
(21) Anmeldenummer: 17207886.7
(22) Anmeldetag: 18.12.2017
(51) Int. Cl.: C08G 64/30, C08G 64/24

(54) **VERFAHREN ZUR HERSTELLUNG EINES POLYCARBONATS UNTER VERWENDUNG EINES ORGANISCHEN LÖSUNGSMITTELS AUF DER GRUNDLAGE VON CHLORKOHLENWASSERSTOFFEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Gegenstand der vorliegenden Erfindung ist ein Verfahren zur Herstellung eines Polycarbonats aus einem Diphenol oder mehreren Diphenolen und einem Diarylcarbonat nach dem Schmelzeumesterungsverfahren. Dabei werden das Diarylcarbonat durch Umsetzung von einem Monophenol mit einem Carbonylhalogenid erhalten. Das erfindungsgemäße Verfahren zeichnet sich dadurch aus, dass es unter Verwendung eines organischen Lösungsmittels auf der Grundlage eines Chlorkohlenwasserstoffs oder mehrerer Chlorkohlenwasserstoffe erfolgt.

## Beschreibung

Gegenstand der vorliegenden Erfindung ist ein Verfahren zur Herstellung eines Polycarbonats aus einem Diphenol oder mehreren Diphenolen und einem Diarylcarbonat nach dem Schmelzeumesterungsverfahren. Dabei werden das Diarylcarbonat durch Umsetzung von einem Monophenol mit einem Carbonylhalogenid erhalten. Das erfindungsgemäße Verfahren zeichnet sich dadurch aus, dass es unter Verwendung eines organischen Lösungsmittels auf der Grundlage eines Chlorkohlenwasserstoffs oder mehrerer Chlorkohlenwasserstoffe erfolgt.

Sowohl Polycarbonate als auch deren Herstellung nach dem Schmelzeumesterungsverfahren sind aus dem Stand der Technik bekannt.

Bei der Herstellung von Polycarbonat durch Schmelzeumesterung werden Dihydroxyarylverbindungen mit Diarylcarbonaten zur Reaktion gebracht, wobei im Sinne einer Umesterungsreaktion die Monohydroxyarylverbindung aus den Diarylcarbonaten abgespalten wird. Die Aufkondensation führt zunächst zur Bildung niedermolekularer Polycarbonat-Oligomere, die unter fortgesetzter Abspaltung der Monohydroxyarylverbindung zu hochmolekularem Polycarbonat weiterreagieren. Der Reaktionsfortschritt kann durch den Einsatz geeigneter Katalysatoren gefördert werden.

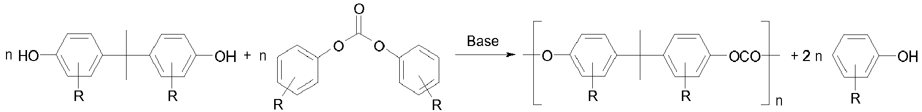

Die Herstellung aromatischer Polycarbonate nach dem Schmelzumesterungsverfahren ist bekannt und beispielsweise beschrieben in "Schnell", Chemistry and Physics of Polycarbonates, Polymer Reviews, Vol. 9, Interscience Publishers, New York, London, Sydney 1964, in D.C. Prevorsek, B.T. Debona and Y. Kersten, Corporate Research Center, Allied Chemical Corporation, Moristown, New Jersey 07960, "Synthesis of Poly(ester)carbonate Copolymers" in Journal of Polymer Science, Polymer Chemistry Edition, Vol. 19, 75-90 (1980), in D. Freitag, U. Grigo, P.R. Müller, N. Nouvertne, BAYER AG, "Polycarbonates" in Encyclopedia of Polymer Science and Engineering, Vol. 11, Second Edition, 1988, Seiten 648-718 und schließlich in Dres. U. Grigo, K. Kircher und P.R. Müller "Polycarbonate" in Becker/Braun, Kunststoff-Handbuch, Band 3/1, Polycarbonate, Polyacetale, Polyester, Celluloseester, Carl Hanser Verlag München, Wien 1992, Seiten 117-299.

Technisch sehr bedeutsam ist die Herstellung von hochmolekularem Polycarbonat aus 2,2-Bis(4-hydroxyphenyl)-propan (kurz auch Bisphenol A oder BPA genannt) und Diphenylcarbonat (kurz auch DPC genannt).

Polycarbonate im Sinne der vorliegenden Erfindung sind sowohl Homopolycarbonate als auch Copolycarbonate und/oder Polyestercarbonate; die Polycarbonate können in bekannter Weise linear oder verzweigt sein. Erfindungsgemäß können auch Mischungen von Polycarbonaten verwendet werden.

Die thermoplastischen Polycarbonate einschließlich der thermoplastischen, aromatischen Polyestercarbonate haben mittlere Molekulargewichte M_{w} (ermittelt durch Messung der relativen Lösungsviskosität bei 25°C in CH₂Cl₂ und einer Konzentration von 0,5 g pro 100 ml CH₂Cl₂) von 20.000 g/mol bis 32.000 g/mol, vorzugsweise von 23.000 g/mol bis 31.000 g/mol, insbesondere von 24.000 g/mol bis 31.000 g/mol.

Ein Teil, bis zu 80 Mol-%, vorzugsweise von 20 Mol-% bis zu 50 Mol-%, der Carbonat-Gruppen in den erfindungsgemäß eingesetzten Polycarbonaten können durch aromatische Dicarbonsäureester-Gruppen ersetzt sein. Derartige Polycarbonate, die sowohl Säurereste der Kohlensäure als auch Säurereste von aromatischen Dicarbonsäuren in die Molekülkette eingebaut enthalten, werden als aromatische Polyestercarbonate bezeichnet. Sie werden im Rahmen der vorliegenden Erfindung unter dem Oberbegriff der thermoplastischen, aromatischen Polycarbonate subsumiert.

Die Herstellung der Polycarbonate erfolgt in bekannter Weise aus Diphenolen, Kohlensäurederivaten, gegebenenfalls Kettenabbrechern und gegebenenfalls Verzweigern, wobei zur Herstellung der Polyestercarbonate ein Teil der Kohlensäurederivate durch aromatische Dicarbonsäuren oder Derivate der Dicarbonsäuren ersetzt wird, und zwar je nach Maßgabe der in den aromatischen Polycarbonaten zu ersetzenden Carbonatstruktureinheiten durch aromatische Dicarbonsäureesterstruktureinheiten.

Für die Herstellung von Polycarbonaten geeignete Dihydroxyarylverbindungen sind solche der Formel (1)

HO-Z-OH (1),

in welcher
- Z: ein aromatischer Rest mit 6 bis 30 C-Atomen ist, der einen oder mehrere aromatische Kerne enthalten kann, substituiert sein kann und aliphatische oder cycloaliphatische Reste bzw. Alkylaryle oder Heteroatome als Brückenglieder enthalten kann.

Bevorzugt steht Z in Formel (2) für einen Rest der Formel (2) 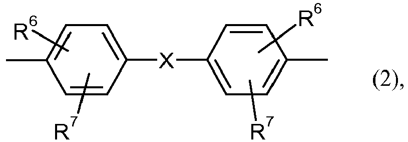 in der
- R⁶ und R⁷: unabhängig voneinander für H, C₁- bis C₁₈-Alkyl-, C₁- bis C₁₈-Alkoxy, Halogen wie Cl oder Br oder für jeweils gegebenenfalls substituiertes Aryl- oder Aralkyl, bevorzugt für H oder C₁- bis C₁₂-Alkyl, besonders bevorzugt für H oder C₁- bis C₈-Alkyl und ganz besonders bevorzugt für H oder Methyl stehen, und
- X: für eine Einfachbindung, -SO₂-, -CO-, -O-, -S-, C₁- bis C₆-Alkylen, C₂- bis Cs-Alkyliden oder C₅- bis C₆-Cycloalkyliden, welches mit C₁- bis C₆-Alkyl, vorzugsweise Methyl oder Ethyl, substituiert sein kann, ferner für C₆- bis C₁₂-Arylen, welches gegebenenfalls mit weiteren Heteroatome enthaltenden aromatischen Ringen kondensiert sein kann, steht.

Bevorzugt steht X für eine Einfachbindung, C₁- bis C₅-Alkylen, C2- bis C₅-Alkyliden, C₅- bis C₆-Cycloalkyliden, -O-, -SO-, -CO-, -S-, -SO₂-
oder für einen Rest der Formel (2a)

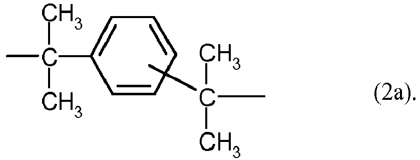

Beispiele für Diphenole (Dihydroxyarylverbindungen) sind: Dihydroxybenzole, Dihydroxydiphenyle, Bis-(hydroxyphenyl)-alkane, Bis-(hydroxyphenyl)-cycloalkane, Bis-(hydroxyphenyl)-aryle, Bis-(hydroxyphenyl)-ether, Bis-(hydroxyphenyl)-ketone, Bis-(hydroxyphenyl)-sulfide, Bis-(hydroxyphenyl)-sulfone, Bis-(hydroxyphenyl)-sulfoxide, 1,1'-Bis-(hydroxyphenyl)-diisopropylbenzole sowie deren kernalkylierte und kernhalogenierte Verbindungen.

Für die Herstellung der erfindungsgemäß zu verwendenden Polycarbonate geeignete Diphenole sind beispielsweise Hydrochinon, Resorcin, Dihydroxydiphenyl, Bis-(hydroxyphenyl)-alkane, Bis(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole sowie deren alkylierte, kernalkylierte und kernhalogenierte Verbindungen.

Bevorzugte Diphenole sind 4,4'-Dihydroxydiphenyl, 2,2-Bis-(4-hydroxyphenyl)-1-phenylpropan, 1,1-Bis-(4-hydroxyphenyl)-phenylethan, 2,2-Bis-(4-hydroxyphenyl)-propan, 2,4-Bis-(4-hydroxyphenyl)-2-methylbutan, 1,3-Bis-[2-(4-hydroxyphenyl)-2-propyl]benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol und 1,1-Bis-(4-hydroxyphenyl)-3,3,5-trimethylcyclohexan (kurz auch Bisphenol TMC genannt).

Besonders bevorzugte Diphenole sind 4,4'-Dihydroxydiphenyl, 1,1-Bis-(4-hydroxyphenyl)-phenyl-ethan, 2,2-Bis-(4-hydroxyphenyl)-propan, 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan.

Diese und weitere geeignete Diphenole sind z.B. in US 2 999 835 A, US 3 148 172 A, US 2 991 273 A, US 3 271 367 A, US 4 982 014 A und US 2 999 846 A, in den deutschen Offenlegungsschriften DE 1 570 703 A, DE 2 063 050 A, DE 2 036 052 A, DE 2 211 956 A und DE 3 832 396 A, der französischen Patentschrift FR 1 561 518 A1, in der Monographie "H. Schnell, Chemistry and Physics of Polycarbonates, Interscience Publishers, New York 1964, S. 28 ff.; S.102 ff.", und in "D.G. Legrand, J.T. Bendler, Handbook of Polycarbonate Science and Technology, Marcel Dekker New York 2000, S. 72ff." beschrieben.

Im Falle der Homopolycarbonate wird nur ein Diphenol eingesetzt, im Falle von Copolycarbonaten werden zwei oder mehr Diphenole eingesetzt. Die verwendeten Diphenole, wie auch alle anderen der Synthese zugesetzten Chemikalien und Hilfsstoffe, können mit den aus ihrer eigenen Synthese, Handhabung und Lagerung stammenden Verunreinigungen kontaminiert sein. Es ist jedoch wünschenswert, mit möglichst reinen Rohstoffen zu arbeiten.

In der gleichen Weise werden eventuell zu verwendende Verzweiger oder Verzweigermischungen der Synthese zugesetzt. Üblicherweise werden Trisphenole, Quarterphenole oder Säurechloride von Tri- oder Tetracarbonsäuren verwendet oder auch Gemische der Polyphenole oder der Säurechloride.

Einige der als Verzweiger verwendbaren Verbindungen mit drei oder mehr als drei phenolischen Hydroxylgruppen sind beispielsweise Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tris-(4-hydroxyphenyl)-benzol, 1,1,1-Tri-(4-hydroxyphenyl)-ethan, Tris-(4-hydroxyphenyl)-phenylmethan, 2,2-Bis-[4,4-bis-(4-hydroxyphenyl)-cyclohexyl] -propan, 2,4-Bis-(4-hydroxyphenyl-isopropyl)-phenol, Tetra-(4-hydroxyphenyl)-methan.

Einige der sonstigen trifunktionellen Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Bevorzugte Verzweiger sind 3,3-Bis-(3-methyl-4-hydroxyphenyl)-2-oxo-2,3-dihydroindol und 1,1,1-Tri-(4-hydroxyphenyl)-ethan.

Die Menge der gegebenenfalls einzusetzenden Verzweiger beträgt 0,05 Mol-% bis 2 Mol-%, bezogen wiederum auf Mole an jeweils eingesetzten Diphenolen, wobei die Verzweiger mit den Diphenolen vorgelegt werden.

Alle diese Maßnahmen zur Herstellung der Polycarbonate sind dem Fachmann geläufig.

Für die Herstellung der Polyestercarbonate geeignete aromatische Dicarbonsäuren sind beispielsweise Orthophthalsäure, Terephthalsäure, Isophthalsäure, tert-Butylisophthalsäure, 3,3'-Diphenyldicarbonsäure, 4,4'-Diphenyldicarbonsäure, 4,4-Benzophenondicarbonsäure, 3,4'-Benzophenondicarbonsäure, 4,4'-Diphenyletherdicarbonsäure, 4,4'-Diphenylsulfondicarbonsäure, 2,2-Bis-(4-carboxyphenyl)-propan, Trimethyl-3-phenylindan-4,5'-dicarbonsäure.

Von den aromatischen Dicarbonsäuren werden besonders bevorzugt die Terephthalsäure und/oder Isophthalsäure eingesetzt.

Derivate der Dicarbonsäuren sind die Dicarbonsäuredihalogenide und die Dicarbonsäuredialkylester, insbesondere die Dicarbonsäuredichloride und die Dicarbonsäuredimethylester.

Der Ersatz der Carbonatgruppen durch die aromatischen Dicarbonsäureestergruppen erfolgt im Wesentlichen stöchiometrisch und auch quantitativ, so dass das molare Verhältnis der Reaktionspartner sich auch im fertigen Polyestercarbonat wiederfindet. Der Einbau der aromatischen Dicarbonsäureestergruppen kann sowohl statistisch als auch blockweise erfolgen.

Diarylcarbonate, die zur Herstellung von Polycarbonaten nach dem Schmelzeumesterungsverfahren eingesetzt werden können, sind Di-C₆-C₁₄-Arylester, vorzugsweise die Diester von Phenol oder substituierten Phenolen, also Diphenylcarbonat oder z.B. Bissalicylcarbonat. Bezogen auf 1 Mol Diphenol werden die Diarylcarbonate in 1,01 bis 1,30 Mol, bevorzugt in 1,02 bis 1,15 Mol eingesetzt.

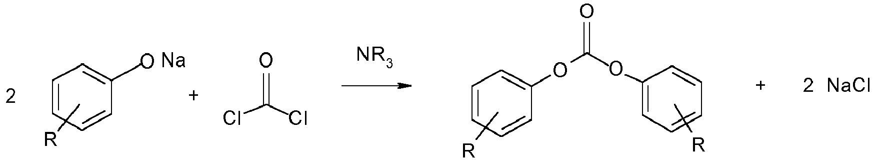

Das ausreagierte, höchstens noch Spuren an (< 2 ppm) Chlorkohlensäurearylestern enthaltende mindestens zweiphasige Reaktionsgemisch lässt man zur Phasentrennung absitzen. Die wässrige alkalische Phase (Reaktionsabwasser) wird abgetrennt und die organische Phase mit verdünnter Salzsäure und Wasser extrahiert. Die vereinigten Wasserphasen werden der Abwasseraufarbeitung zugeführt, wo Lösungsmittel- und Katalysatoranteile durch Strippen oder Extraktion abgetrennt und rückgeführt werden. Anschließend werden nach der Einstellung eines bestimmten pH-Wertes von z.B. 6 bis 8, z.B. durch Salzsäurezugabe, die gegebenenfalls noch verbleibenden organischen Verunreinigungen wie z.B. Monophenol durch Behandlung mit Aktivkohle entfernt und der Chloralkalielektrolyse zugeführt.

In einer anderen Variante der Aufarbeitung wird das Reaktionsabwasser nicht mit den Waschphasen vereinigt, aber nach Strippen oder Extraktion zur Abtrennung von Lösungsmitteln und Katalysatorresten, auf einem bestimmten pH-Wert von z.B. 6 bis 8, z.B. durch Salzsäurezugabe eingestellt und nach Abtrennung der noch verbleibenden organischen Verunreinigungen wie z.B. Monophenol durch Behandlung mit Aktivkohle der Chloralkalielektrolyse zugeführt.

Die Waschphasen können nach Entfernung der Lösungsmittel- und Katalysatoranteile durch Strippen oder Extraktion gegebenenfalls wieder der Synthese zugeführt werden.

Die Herstellung der Diarylcarbonate erfolgt gemäß dem Stand der Technik durch Reaktion von Monophenolen und einem Carbonylhalogenid in einem inerten Lösungsmittel in Gegenwart von Alkali und einem Stickstoffkatalysator nach dem Phasengrenzflächenverfahren unter Bildung des Alkalihalogenids, z.B. NaCl. Das Carbonylhalogenid kann ein dabei ein Carbonyldihalogenid, vorzugsweise Phosgen, oder Disphosgen oder Triphosgen sein; insbesondere ist Phosgen bevorzugt. Optimierungen des Verfahrens durch Verbesserung der Durchmischung und Einhaltung eines engen Temperatur- und pH-Profils als auch Isolierung des Diarylcarbonates werden in EP 1 219 589 A1, EP 1 216 981 A2, EP 1 216 982 A2 und EP 784 048 A1 beschrieben.

Besonders geeignete Monophenole zur Herstellung der Diarylcarbonate in Schritt c) sind Phenole der Formel (II) 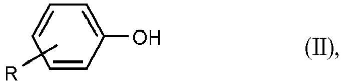 worin
- R: Wasserstoff, Halogen oder ein verzweigter oder unverzweigter C₁- bis C₉-Alkylrest oder Alkoxycarbonylrest ist.

Bevorzugt sind also Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol und 2,4,6-Tribromphenol oder Salicylsäuremethylester. Besonders bevorzugt ist Phenol.

Das eingesetzte Alkali zur Bildung des Phenolats kann eine Alkalilauge mit Hydroxiden aus der Reihe: Na-, K-, Li-Hydroxid sein, bevorzugt ist Natronlauge, und wird im neuen Verfahren vorzugsweise als 10 bis 55 Gew.%-ige Lösung eingesetzt.

Die Herstellung der Diarylcarbonate kann durch Katalysatoren, wie tertiäre Amine, N-Alkylpiperidine oder Oniumsalze beschleunigt werden. Bevorzugt werden Tributylamin, Triethylamin und N-Ethylpiperidin verwendet.

Der eingesetzte Amin-Katalysator kann offenkettig oder zyklisch sein, besonders bevorzugt sind Triethylamin und Ethylpiperidin. Der Katalysator wird vorzugsweise als 1 bis 55 Gew.%-ige Lösung eingesetzt.

Unter Oniumsalzen werden hier Verbindungen wie NR₄X verstanden, wobei R ein Alkyl und/oder Arylrest und/oder ein H sein kann und X ein Anion ist.

Das Carbonylhalogenid, insbesondere Phosgen, kann flüssig, gasförmig oder gelöst in einem inerten Lösungsmittel eingesetzt werden.

Die Herstellung von Phosgen aus Kohlenmonoxid und Chlor in ist an sich bekannt, beispielsweise aus EP 881 986 B1 oder EP 1 640 341 A2. Die Umsetzung des Kohlenmonoxids erfolgt durch Umsetzung des Kohlenmonoxids mit Chlor zu Phosgen, zum Beispiel an einem Aktivkohle-Katalysator. Alternativkatalysatoren können aber auch eingesetzt werden. Hier kann auf den Stand der Technik verwiesen werden (z.B. DE 332 72 74 A1; GB 583 477 A; WO 97/30932 A1; WO 96/16898 A1; US 6,713,035 B1). Im technischen Maßstab wird Phosgen überwiegend durch Umsetzung von Kohlenmonoxid mit Chlor, bevorzugt an Aktivkohle als Katalysator hergestellt. Die stark exotherme Gasphasenreaktion erfolgt bei Temperaturen von mindestens 100°C bis maximal 600°C in der Regel in Rohrbündelreaktoren. Die Abführung der Reaktionswärme kann auf unterschiedliche Weise erfolgen, beispielsweise durch ein flüssiges Wärmetauschmittel, wie z.B. in WO 03/072237 A1 beschrieben, oder durch Siedekühlung über einen Sekundärkreislauf unter gleichzeitiger Nutzung der Reaktionswärme zur Dampferzeugung, wie z.B. in US 4,764,308 A offenbart. Bevorzugt wird Aktivkohle als Katalysator eingesetzt, wobei die Aktivkohle einen Gehalt an Gesamtschwefel von kleiner 1 Gew-%, insbesondere kleiner 0.5 Gew.-% bezogen auf das Gesamtgewicht des Katalysators enthält. Weiterhin wird die Herstellung von Phosgen bevorzugt bei Temperaturen kleiner gleich 300°C durchgeführt und weiterhin wird die Herstellung von Phosgen mit der Erzeugung von Dampf kombiniert. Weiterhin bevorzugt hat der Katalysator eine spezifische Oberfläche von größer 10 m2/g.

Gemäß dem Stand der Technik vorzugsweise verwendbare inerte Lösungsmittel für die Herstellung von Diarylcarbonaten, die für die Herstellung von Polycarbonaten verwendet werden können, sind Lösungsmittel auf der Grundlage von Chlorkohlenwasserstoffen wie beispielsweise Dichlormethan, Toluol, den verschiedenen Dichlorethanen oder Chlorpropanverbindungen, Chlorbenzol oder Chlortoluol, oder Gemischen aus zwei oder mehr dieser Chlorkohlenwasserstoffe. Vorzugsweise wird ein inertes Lösungsmittel auf der Grundlage von Dichlormethan oder einem Gemisch aus Dichlormethan und Chlorbenzol eingesetzt. Im Rahmen der vorliegenden Erfindung werden solche inerten Lösungsmittel oder Gemische von inerten Lösungsmitteln zusammenfassend in der Einzahl "organisches Lösungsmittel" und in der Mehrzahl "organische Lösungsmittel" genannt.

Ein Nachteil der Verfahren aus dem Stand der Technik ist, dass für die Herstellung der Diarylcarbonate ein organisches Lösungsmittel eingesetzt werden muss, dessen Gehalt an Verunreinigungen sehr gering ist, das also von hoher Reinheit ist, um Polycarbonate erhalten zu können, die den Qualitätsanforderungen entsprechen.

Die von der Reinheit des organischen Lösungsmittels abhängigen Qualitätsanforderungen sind beispielsweise optische Eigenschaften wie der Yellowness Index (YI), der visuelle Eindruck und eine normgerechte relative Lösungsviskosität.

Insbesondere herrscht im Stand der Technik die Meinung vor, dass das Vorhandensein von Tetrachlormethan die optischen Eigenschaften von Polycarbonaten verschlechtert. Dem entsprechend soll der Gehalt von Tetrachlormethan bei der Herstellung von Diarylcarbonaten und Polycarbonaten aus diesen Diarylcarbonaten möglichst niedrig gehalten werden.

So offenbart WO 2015 / 119981 A2, dass der Gehalt an Tetrachlormethan im zur Herstellung der Diarylcarbonate verwendeten Phosgen maximal 10 ppm betragen dürfe.

Auch der Gehalt an Chlorethan im organischen Lösungsmittel zur Herstellung von Diarylcarbonaten und Polycarbonaten wird hinsichtlich der optischen Eigenschaften der erhaltenen Polycarbonate kritisch gesehen.

Organische Lösungsmittel mit den erforderlichen hohen Reinheiten zur Verfügung zu stellen, ist aber aufwändig und teuer. So müssen diese organischen Lösungsmittel vor dem Einsatz aufwändig gereinigt oder mit speziellen Verfahren hergestellt werden, organische Lösungsmittel höherer Qualität eingesetzt oder bei Verunreinigung öfter erneuert oder zumindest gereinigt werden. Diese Reinigung kann beispielsweise durch Destillation geschehen, was einen hohen Energieverbrauch bedingt. Die Verunreinigungen müssen dann in speziellen Anlagen entsorgt werden, beispielsweise durch Verbrennen, was ebenfalls aufwändig und teuer ist, insbesondere da dabei Umweltbelastungen zu vermeiden sind. Auch führt es häufig zu teuren Betriebsstillständen, wenn auf Grund von Verunreinigungen im organischen Lösungsmittel dieses ausgetauscht werden muss.

Aufgabe der vorliegenden Erfindung ist es daher, ein Verfahren zur Herstellung von Polycarbonat zur Verfügung zu stellen, bei dem bei der Umsetzung eines in der wässrigen Phase gelösten Monophenols oder mehrerer in der wässrigen Phase gelösten Monophenole mit einem in einem organischen Lösungsmittel gelösten Carbonylhalogenid zu einem in dem organischen Lösungsmittel gelösten Diarylcarbonat oder mehreren in dem organischen Lösungsmittel gelösten Diarylcarbonaten ein organisches Lösungsmittel eingesetzt werden kann, das einen erhöhten Gehalt an Verunreinigungen, insbesondere einen erhöhten Gehalt an Tetrachlorkohlenstoff und/oder Chlorethan, aufweist, ohne dass sich die optischen und rheologischen Eigenschaften des Polycarbonats verschlechtern. Insbesondere sollen sich der Yellowness Index, der visuelle Eindruck, und die relative Lösungsviskosität des Polycarbonats nicht verschlechtern. Dabei soll bevorzugt der YI-Wert nicht höher als 2,5, besonders bevorzugt nicht höher als 2 sein, ganz besonders bevorzugt nicht höher als 1,7. Der durch Inaugenscheinnahme geprüfte visuelle Eindruck von Proben von erfindungsgemäß hergestelltem Polycarbonat soll gleich gut wie der von Proben von nach dem Stand der Technik hergestellten Polycarbonat sein. Außerdem soll die relative Lösungsviskosität innerhalb des normgerechten Bereichs liegen.

Auch ist es Aufgabe der vorliegenden Erfindung, ein Verfahren zur Herstellung von Polycarbonat zur Verfügung zu stellen, bei dem Betriebsstillstände, die zum Austausch des organischen Lösungsmittels dienen, im Vergleich zum Stand der Technik weniger häufig vorgenommen werden müssen, oder bei dem das organische Lösungsmittel während des Verfahrens nicht aufwändig gereinigt werden muss, oder bei dem das organische Lösungsmittel während des Verfahrens nicht teilweise entnommen und durch eines mit einem verringerten Gehalt an Verunreinigungen, insbesondere einem verringerten Gehalt an Tetrachlorkohlenstoff und/oder Chlorethan, ersetzt werden muss.

Es wurde überraschend festgestellt, dass die Aufgabe durch ein Verfahren gemäß Anspruch 1 gelöst wird.

Insbesondere wird die Aufgabe dadurch gelöst, dass bei der Herstellung von Diarylcarbonaten aus Carbonylhalogeniden und Monophenolen und weiter von Polycarbonaten aus diesen Diarylcarbonaten ein organisches Lösungsmittel auf der Grundlage eines Chlorkohlenwasserstoffs oder mehrerer Chlorkohlenwasserstoffe eingesetzt wird, das einen Gehalt an Tetrachlormethan von 0,05 bis 7 Gew.-% und einen Gehalt an Chlorethan von 0,3 bis 10 Gew. % aufweist.

Bevorzugt ist das organische Lösungsmittel eines auf der Grundlage von Chlorkohlenwasserstoffen ausgewählt aus der Reihe umfassend Methylenchlorid, Chlorbenzol, Chloroform oder Mischungen aus diesen chlorierten Kohlenwasserstoffen.

Außerdem enthält das erfindungsgemäße organische Lösungsmittel noch ein oder mehrere sonstige Komponenten, auch als Nebenkomponenten bezeichnet. Die Nebenkomponenten sind ohne Anspruch auf Vollständigkeit beispielsweise Wasser, Toluol, Styrol, Methanol, Benzol, o-Xylol, oder Tetrachlorethen.

Im Einzelnen umfasst das erfindungsgemäße Verfahren zur Herstellung von Polycarbonaten nach dem Schmelzumesterungsverfahren wenigstens folgende Schritte:
(a) Lösen eines Monophenols in einer wässriger Phase und Lösen eines Carbonylhalogenids in einem organischen Lösungsmittel,
(b) Umsetzung des in der wässrigen Phase gelösten Monophenols mit dem in dem organischen Lösungsmittel gelösten Carbonylhalogenid zu einem in dem organischen Lösungsmittel gelösten Diarylcarbonat,
(c) Zuführung dieses in dem organischen Lösungsmittel gelösten Diarylcarbonats zu Schritt (d),
(d) Abtrennung des organischen Lösungsmittel von dem oder den in Schritt (b) erhaltenen einen Diarylcarbonats und Zuführung dieses Diarylcarbonats zu Schritt (e) und Rückführung des organischen Lösungsmittels zu Schritt (a),
(e) Umsetzung eines oder mehrerer Diphenole mit dem Diarylcarbonat aus Schritt (d) zu einem Polycarbonat und einem Monophenol oder mehreren Monophenolen, ,
wobei das organische Lösungsmittel folgende Komponenten aufweist:

| | |
|---|---|
| Methylenchlorid | 0 bis 99,65 Gew.-%, |
| Chlorbenzol | 0 bis 99,65 Gew-% |
| Chloroform | 0 bis 99,65 Gew-% |
| Chlorethan | 0,3 bis 10 Gew.-%, |
| Tetrachlormethan | 0,05 bis 7,0 Gew.-%, |
| sonstige Komponenten | 0 bis 2,0 Gew.-%, |

wobei die Summe der Gehalte von Methylenchlorid, Chlorbenzol und Chloroform mindestens 81,00 Gew.-% und höchstens 99,65 Gew.-% beträgt, und wobei Summe der Gehalte von Methylenchlorid, Chlorbenzol und Chloroform vermehrt um die Summe der Gehalte von Chlorethan, Tetrachlormethan und der sonstigen Komponenten 100 Gew.-% beträgt. Dabei ist in dem organischen Lösungsmittel immer mindestens eine Komponente ausgewählt aus den Komponenten Methylenchlorid, Chlorbenzol und Chloroform enthalten.

Die Summe der Gehalte von Chlorethan, Tetrachlormethan und sonstigen Komponenten im organischen Lösungsmittel beträgt also mindestens 0,35 Gew.-% und höchstens 19,0 Gew.-%.

Erfindungsgemäß bevorzugt beträgt der Gehalt an Methylenchlorid gegenüber der Summe der Gehalte von Chlorbenzol und Chloroform wenigstens 98,0 Gew.-% bis 99,999 Gew.-%, bevorzugt 99,0 Gew.% bis 99,995 Gew.-%.

Alternativ erfindungsgemäß bevorzugt beträgt die Summe der Gehalte an Methylenchlorid und Chlorbenzol gegenüber dem Gehalt von Chloroform wenigstens 98,0 Gew.-% bis 99,999 Gew.-%, bevorzugt 99,0 Gew.% bis 99,995 Gew.-%.

Dabei ist das Verhältnis des Gehalts an Methylenchlorid gegenüber dem Gehalt an Chlorbenzol von 40 Gew.-% Methylenchlorid zu 60 Gew.-% Chlorbenzol bis 60 Gew.-% Methylenchlorid zu 40 Gew.-% Chlorbenzol,
bevorzugt 45 Gew.-% Methylenchlorid zu 55 Gew.-% Chlorbenzol bis 55 Gew.-% Methylenchlorid zu 45 Gew.-% Chlorbenzol,
besonders bevorzugt 48 Gew.-% Methylenchlorid zu 52 Gew.-% Chlorbenzol bis 52 Gew.-% Methylenchlorid zu 48 Gew.-% Chlorbenzol,
ganz besonders bevorzugt 49 Gew.-% Methylenchlorid zu 51 Gew.-% Chlorbenzol bis 51 Gew.-% Methylenchlorid zu 49 Gew.-% Chlorbenzol,
insbesondere 50 Gew.-% Methylenchlorid zu 50 Gew.-% Chlorbenzol.

Außerdem erfindungsgemäß bevorzugt beträgt der Gehalt von Chlorethan im organischen Lösungsmittel von 0,9 Gew.-% bis 8,0 Gew.-%, bevorzugt von 2,0 Gew-% bis 7,0 Gew.-%, besonders bevorzugt von 2,5 Gew.-% bis 6,0.

Zusätzlich erfindungsgemäß bevorzugt beträgt der Gehalt von Tetrachlormethan im organischen Lösungsmittel von 0,2 Gew.-% bis 7,0 Gew.-%, bevorzugt von 0,5 bis 6,0 Gew.-%, besonders bevorzugt von 1,0 Gew.-% bis 4,0 Gew.-%.

Insbesondere erfindungsgemäß bevorzugt beträgt im organischen Lösungsmittel der Gehalt von Chlorethan von 0,9 Gew.-% bis 8,0 Gew.-% und der Gehalt von Tetrachlormethan von 0,2 Gew.-% bis 7,0 Gew.-%,
bevorzugt der Gehalt von Chlorethan von 2,0 Gew-% bis 7,0 Gew.-% und der von Tetrachlormethan von 0,5 bis 6,0 Gew.-%,
besonders bevorzugt der Gehalt von Chlorethan von 2,5 Gew.-% bis 6,0 Gew.-% und der von Tetrachlormethan von 1,0 Gew.-% bis 4,0 Gew.-%.

Für alle vorgenannten Fälle gilt, dass die Summe der Gehalte von Methylenchlorid, Chlorbenzol und Chloroform vermehrt um die Summe der Gehalte von Chlorethan, Tetrachlormethan und der sonstigen Komponenten 100 Gew.-% beträgt.

Das erfindungsgemäße Verfahren zeichnet sich weiterhin dadurch aus, dass in Schritt (d) das organische Lösungsmittel durch Destillation abgetrennt wird.

Das erfindungsgemäße Verfahren zeichnet sich weiterhin dadurch aus, dass das in Schritt (e) erhaltene Monophenol wieder in Schritt (a) eingesetzt wird.

Das erfindungsgemäße Verfahren zeichnet sich weiterhin dadurch aus, das organische Lösungsmittel nach der Abtrennung in Schritt (d) ohne weitere Abtrennung von flüssigen oder gasförmigen Bestandteilen wieder Schritt (a) zuführt wird.

Das erfindungsgemäße Verfahren zeichnet sich weiterhin dadurch aus, dass das organische Lösungsmittel in einem in Bezug auf dieses Lösungsmittel geschlossenen Kreislauf geführt wird. Dabei wird die Masse des Lösungsmittels während der Durchführung des Verfahrens nicht vermehrt, vorzugsweise konstant gehalten.

Erfindungsgemäß bevorzugt werden erst nach einer zeitlichen Periode statt, in der pro Massenanteil des organischen Lösungsmittels 1000 bis 15.000, bevorzugt 1.500 bis 12.000, besonders bevorzugt 1.750 bis 8.000, ganz besonders bevorzugt 2.000 bis 6.000 Massenanteiledes Diarylcarbonats gemäß den Schritten (a) bis (d) hergestellt werden, 100 % oder weniger, bevorzugt 70 % oder weniger, besonders bevorzugt 50 % oder weniger, ganz besonders bevorzugt 30 % oder weniger des organischen Lösungsmittels ersetzt oder aufgereinigt.

Dadurch kann die Zeit zwischen zwei Betriebsstillständen zum Austausch des organischen Lösungsmittels gegenüber dem Stand der Technik mehr als verdoppelt werden.

Alternativ erfindungsgemäß bevorzugt oder zusätzlich wird innerhalb einer zeitlichen Periode, in der pro Massenanteil des organischen Lösungsmittels 1000 bis 15.000, bevorzugt 1.500 bis 12.000, besonders bevorzugt 1.750 bis 8.000, ganz besonders bevorzugt 2.000 bis 6.000 Massenanteile des Diarylcarbonats gemäß den Schritten (a) bis (d) hergestellt werden, maximal ein Anteil von 20 %, bevorzugt maximal ein Anteil von 10 Gew.-%, besonders bevorzugt maximal ein Anteil von 5 Gew.-%, ganz besonders bevorzugt maximal ein Anteil von 2 Gew.-%, insbesondere bevorzug maximal ein Anteil von 1 Gew.-%, des organischen Lösungsmittels dem Lösungsmittel entnommen wird und gleichzeitig durch eine dem entnommenen Anteil an organischem Lösemittel einsprechende Menge eines Lösungsmittelgemischs umfassend ein oder mehrere Komponenten ausgewählt aus Methylenchlorid, Chlorbenzol und Chloroform ergänzt. Dabei entspricht erfindungsgemäß bevorzugt das Mischungsverhältnis von Methylenchlorid zu Chlorbenzol zu Chloroform des Lösungsmittelgemischs, das wieder hinzugefügt wird, dem Mischungsverhältnis von Methylenchlorid zu Chlorbenzol zu Chloroform des organischen Lösungsmittels, das zu Beginn der zeitlichen Periode vorlag.

Auf diese Weise wird die Zeit zwischen zwei Betriebsstillständen im gegenüber dem Stand der Technik einerseits nochmals vergrößert, andererseits die Menge an ausgetauschten verunreinigtem organischen Lösungsmittel sehr klein gehalten.

Das erfindungsgemäße Verfahren ist in einer bevorzugten Variante dadurch gekennzeichnet, dass das organische Lösungsmittel nach Abtrennung in Schritt (d) ohne weitere Aufreinigung wieder Schritt (a) zuführt wird. Insbesondere wird das organische Lösungsmittel nicht einer Aufreinigung durch eine weitere Destillation zur Abtrennung von flüssigen oder gasförmigen Bestandteilen, insbesondere von Tetrachlormethan oder Chlorethan, oder einem anderen Verfahren zur Abtrennung von flüssigen oder gasförmigen Bestandteilen, insbesondere von Tetrachlormethan oder Chlorethan, unterworfen. Möglich ist jedoch eine Reinigung des organischen Lösungsmittels von Feststoffen durch Siebe oder Filter.

Der Verzicht auf eine weitere destillative Abtrennung ermöglicht es auch, geringere Mengen von Wärmeträgern in das organische Lösungsmittel zu geben, die zur Vermeidung sogenannter Hot Spots dienen. Dies führt zu einer weiteren Vereinfachung und Verbilligung des Verfahrens.

Alternativ oder zusätzlich ist das erfindungsgemäße Verfahren weiter dadurch gekennzeichnet, dass in Schritt (a) der molare Überschuss des Carbonylhalogenids gegenüber dem molaren Anteil des einen Monophenols oder der Summe der molaren Anteile der mehreren Monophenole von 2 bis 10 %, bevorzugt von 2,5 bis 4,5 %, besonders bevorzugt von 3 bis 4 % beträgt.

Alternativ oder zusätzlich ist das erfindungsgemäße Verfahren dadurch gekennzeichnet, dass in Schritt (a) das Gehalt des Monophenols oder die Summe der Gehalte der Monophenole in der wässrigen Phase von 20 bis 40 Gew.-%, bevorzugt 25 bis 35 Gew.-%, besonders bevorzugt 28 bis 32 Gew.-% beträgt.

Alternativ oder zusätzlich ist das erfindungsgemäße Verfahren dadurch gekennzeichnet, dass es sich bei der wässrigen Phase um Alkali- oder Erdalkalilauge handelt.

Alternativ oder zusätzlich ist das erfindungsgemäße Verfahren dadurch gekennzeichnet, dass der pH-Wert in Schritt (a) in der wässrigen Phase 9,0 bis 11,0, bevorzugt 9,5 bis 10,5, besonders bevorzugt 9,8 bis 10,2 beträgt.

Alternativ oder zusätzlich ist das erfindungsgemäße Verfahren dadurch gekennzeichnet, dass in Schritt (c) der Gehalt des Diarylcarbonats in dem organischen Lösungsmittel von 30 bis 50 Gew.-%, bevorzugt 35 bis 45 Gew.-%, besonders bevorzugt 38 bis 42 Gew.-% beträgt.

Alternativ oder zusätzlich ist das erfindungsgemäße Verfahren dadurch gekennzeichnet, dass das Carbonylhalogenid ausgewählt ist aus der Reihe umfassend: ein Carbonyldihalogenid, Diphosgen, Triphosgen, bevorzugt ein Carbonyldihalogenid, besonders bevorzugt Phosgen.

Die nach dem erfindungsgemäßen Verfahren hergestellten Polycarbonate weisen eine genauso gute Eigenfarbe der durch Spritzguss aus diesen Polycarbonaten hergestellten Formkörper aus wie die Eigenfarbe von Spritzkörpern aus Polycarbonaten, die mit Verfahren nach den Stand der Technik hergestellt wurden. Diese Eigenfarbe der transparenten spritzfrischen Formkörper ist gekennzeichnet durch einen sehr geringen Gelbwert, der durch den sog. "Yellowness-Index" (Y.I.) nach ASTM E313 an spritzgegossenen Platten definierter Dicke bestimmt wird. Alle Angaben zum Y.I. der Polycarbonate beziehen sich auf diese Bestimmungsmethode. Die YI-Werte der erfindungsgemäß hergestellten Polycarbonate liegen maximal bei 2,0, vorzugsweise maximal bei 1,7.

Auch weisen die nach dem erfindungsgemäßen Verfahren hergestellten Polycarbonate genauso gute und normgerechte relative Lösungsviskositäten auf wie mit Verfahren nach dem Stand der Technik hergestellte Polycarbonate. Diese relativen Lösungsviskositäten liegen normgerecht im Bereich von 15 bis 40, bevorzugt von 20 bis 35. Die relative Lösungsviskosität wurde dabei ermittelt durch Messung mit einem Lauda Ubbelohde Viskosimeter Typ Proline PV24 bei 25°C in CH₂Cl₂ und einer Konzentration von 0,5 g Polycarbonat pro 100 ml CH₂Cl₂.

Weiterer Gegenstand der vorliegenden Erfindung ist daher auch ein Polycarbonat, dass noch dem erfindungsgemäßen Verfahren hergestellt wurde. Dieses Polycarbonat zeichnet sich unter anderem dadurch aus, dass es einen YI-Wert von maximal 2,0, bevorzugt von maximal 1,7 aufweist. Auch zeichnet sich das erfindungsgemäße Polycarbonat dadurch aus, dass es relative Lösungsviskositäten von 15 bis 40, bevorzugt von 20 bis 35 aufweist.

Weiterer Gegenstand der Erfindung ist auch ein organisches Lösungsmittel, dass für die Durchführung des erfindungsgemäßen Verfahrens geeignet ist. Dieses organische Lösungsmittel weist folgende Komponenten auf:

| | |
|---|---|
| Methylenchlorid | 0 bis 99,65 Gew.-%, |
| Chlorbenzol | 0 bis 99,65 Gew-% |
| Chloroform | 0 bis 99,65 Gew-% |
| Chlorethan | 0,3 bis 10 Gew.-%, |
| Tetrachlormethan | 0,05 bis 7,0 Gew.-%, |
| sonstige Komponenten | 0 bis 2,0 Gew.-%, |

wobei die Summe der Gehalte von Methylenchlorid, Chlorbenzol und Chloroform mindestens 81,00 Gew.-% und höchstens 99,65 Gew.-% beträgt,
und wobei Summe der Gehalte von Methylenchlorid, Chlorbenzol und Chloroform vermehrt um die Summe der Gehalte von Chlorethan, Tetrachlormethan und der sonstigen Komponenten 100 Gew.-% beträgt. Dabei ist in dem organischen Lösungsmittel immer mindestens eine Komponente ausgewählt aus den Komponenten Methylenchlorid, Chlorbenzol und Chloroform enthalten.

Die Summe der Gehalte von Chlorethan, Tetrachlormethan und sonstigen Komponenten im organischen Lösungsmittel beträgt also mindestens 0,35 Gew.-% und höchstens 19,0 Gew.-%.

Erfindungsgemäß bevorzugt beträgt der Gehalt an Methylenchlorid gegenüber der Summe der Gehalte von Chlorbenzol und Chloroform wenigstens 98,0 Gew.-% bis 99,999 Gew.-%, bevorzugt 99,0 Gew.% bis 99,995 Gew.-%.

Alternativ erfindungsgemäß bevorzugt beträgt die Summe der Gehalte an Methylenchlorid und Chlorbenzol gegenüber dem Gehalt von Chloroform wenigstens 98,0 Gew.-% bis 99,999 Gew.-%, bevorzugt 99,0 Gew.% bis 99,995 Gew.-%.

Dabei ist das Verhältnis des Gehalts an Methylenchlorid gegenüber dem Gehalt an Chlorbenzol von 40 Gew.-% Methylenchlorid zu 60 Gew.-% Chlorbenzol bis 60 Gew.-% Methylenchlorid zu 40 Gew.-% Chlorbenzol,
bevorzugt 45 Gew.-% Methylenchlorid zu 55 Gew.-% Chlorbenzol bis 55 Gew.-% Methylenchlorid zu 45 Gew.-% Chlorbenzol,
besonders bevorzugt 48 Gew.-% Methylenchlorid zu 52 Gew.-% Chlorbenzol bis 52 Gew.-% Methylenchlorid zu 48 Gew.-% Chlorbenzol,
ganz besonders bevorzugt 49 Gew.-% Methylenchlorid zu 51 Gew.-% Chlorbenzol bis 51 Gew.-% Methylenchlorid zu 49 Gew.-% Chlorbenzol,
insbesondere 50 Gew.-% Methylenchlorid zu 50 Gew.-% Chlorbenzol.

Außerdem erfindungsgemäß bevorzugt beträgt der Gehalt von Chlorethan im organischen Lösungsmittel von 0,9 Gew.-% bis 8,0 Gew.-%, bevorzugt von 2,0 Gew-% bis 7,0 Gew.-%, besonders bevorzugt von 2,5 Gew.-% bis 6,0.

Zusätzlich erfindungsgemäß bevorzugt beträgt der Gehalt von Tetrachlormethan im organischen Lösungsmittel von 0,2 Gew.-% bis 7,0 Gew.-%, bevorzugt von 0,5 bis 6,0 Gew.-%, besonders bevorzugt von 1,0 Gew.-% bis 4,0 Gew.-%.

Insbesondere erfindungsgemäß bevorzugt beträgt im organischen Lösungsmittel der Gehalt von Chlorethan von 0,9 Gew.-% bis 8,0 Gew.-% und der Gehalt von Tetrachlormethan von 0,2 Gew.-% bis 7,0 Gew.-%,
bevorzugt der Gehalt von Chlorethan von 2,0 Gew-% bis 7,0 Gew.-% und der von Tetrachlormethan von 0,5 bis 6,0 Gew.-%,
besonders bevorzugt der Gehalt von Chlorethan von 2,5 Gew.-% bis 6,0 Gew.-% und der von Tetrachlormethan von 1,0 Gew.-% bis 4,0 Gew.-%.

Für alle vorgenannten Fälle gilt, dass die Summe der Gehalte von Methylenchlorid, Chlorbenzol und Chloroform vermehrt um die Summe der Gehalte von Chlorethan, Tetrachlormethan und der sonstigen Komponenten 100 Gew.-% beträgt.

Ein solches organisches Lösungsmittel ist ohne aufwendige Reinigung verfügbar und billiger als organische Lösungsmittel mit geringeren Gehalten an Chlorethan oder Tetrachlormethan. Es eignet sich aber genauso wie organische Lösungsmittel mit geringeren Gehalten an Chlorethan oder Tetrachlormethan für die Herstellung von Polycarbonaten mit guten optischen Eigenschaften, insbesondere von Polycarbonaten mit einem YI-Wert von maximal 2,0, bevorzugt von maximal 1,7.

Die vorliegende Erfindung wird nachfolgend anhand von Beispielen verdeutlich, ohne dass die Erfindung auf diese Beispiele eingeschränkt werden soll.

Die hier beschriebenen Versuche wurden in einer kontinuierlich betriebenen Anlage zur Herstellung von Polycarbonat nach dem Schmelzeumesterungsverfahren durchgeführt. Die angegebenen Unterdrücke sind Absolutdrücke.

### Beispiel 1

### a) Herstellung und Aufarbeitung von Diphenylcarbonat (DPC)

In einen senkrecht stehenden, gekühlten Rohrreaktor wurde kontinuierlich ein Gemisch aus 145,2 kg/h 14,5 %-iger Natronlauge, hergestellt durch Verdünnung von 65,8 kg/h einer 32,0 %-igen Natronlauge mit 79,4 kg/h vollentsalztem Wasser (VE-Wasser), und 48,3 kg/h frisches und/oder zurückgewonnenes Phenol mit 86,2 kg/h eines organischen Lösungsmittels und 27,5 kg/h Phosgen (8 Mol % Überschuss bzgl. auf Phenol) zusammengebracht. Dieses Reaktionsgemisch wurde auf eine Temperatur von 33°C gekühlt und nach einer mittleren Verweilzeit von 15 Sekunden ein pH-Wert von 11,5 gemessen. Zu diesem Reaktionsgemisch wurden nun in einer zweiten Stufe des Verfahrens 5,4 kg/h 50,0 %-iger NaOH zudosiert, so dass der pH-Wert der zweiten Reaktionsstufe nach einer weiteren Verweilzeit von 5 Minuten 8,5 beträgt. In der zweiten Stufe des Verfahrens wurde das Reaktionsgemisch durch das Durchleiten eines mit Verengungen versehenen Rohres ständig gemischt. Die Reaktionstemperatur wurde nach erneuter Zugabe der NaOH durch Kühlen auf 30°C eingestellt. Nach Abtrennung der organischen von der wässrigen Phase (Reaktionsabwasser) wurde die erhaltene Diphenylcarbonat-Lösung mit 0,6 %-iger Salzsäure und Wasser gewaschen. Nach Entfernung des Lösungs-mittels wurde 55 kg/h 99,9 %-iges Diphenylcarbonat erhalten. Das Reaktionsabwasser wurde nicht mit den Waschphasen vereinigt und wurde durch Strippen mit Wasserdampf von Lösungsmittelresten und Katalysator befreit. Nach Neutralisierung (pH 7) mit Salzsäure und Behandlung mit Aktivkohle enthielt das Reaktionsabwasser 17,0 % NaCl und < 2 ppm Phenol.

Das oben beschriebene Verfahren wird nacheinander jeweils mit organischen Lösungsmitteln unterschiedlicher Zusammensetzung durchgeführt. Das organische Lösungsmittel hat jeweils die in Tabelle 1 aufgeführte Zusammensetzung.

### b) Herstellung von Polycarbonat

Aus einer Vorlage wurden 107 kg/h Schmelzegemisch, bestehend aus 55 kg/h Diphenylcarbonat (257 mol/h) und 52 kg/h Bisphenol A (227 mol/h), unter Zusetzen von 6.5 g des Phenoladdukts von Tetraphenylphosphoniumphenolat mit 65,5 % Tetraphenylphosphoniumphenolat/h (0,10 mmol/h; das sind 0,0043 Mol-%) gelöst in 55.9 g Phenol/h durch einen Wärmetauscher gepumpt, auf 190°C erwärmt und durch eine Verweilkolonne bei 12 bar und 190°C geführt. Die mittlere Verweilzeit betrug 50 Minuten.

Die Schmelze wurde dann über ein Entspannungsventil in einen unter 200 mbar stehenden Abscheider geleitet. Die abfließende Schmelze wurde in einem, ebenfalls unter 200 mbar stehenden, Fallfilmverdampfer wieder auf 189°C erwärmt und in einer Vorlage aufgefangen. Nach einer Verweilzeit von 20 Minuten wurde die Schmelze in die nächsten drei, gleichartig aufgebauten Stufen gepumpt. Die Bedingungen in der 2. / 3. / 4. Stufe waren 100/74/40 mbar, 218 / 251 / 276°C und 20 Minuten / 10 Minuten / 10 Minuten. Das entstandene Oligomere hatte eine relative Lösungsviskosität von 1,09. Alle Brüden wurden über Druckregelungen in eine unter Vakuum stehende Kolonne geführt und als Kondensate abgeleitet.

Danach wurde das Oligomer in einem sich anschließenden Korbreaktor bei 278°C und 3,0 mbar bei einer Verweilzeit von 45 Minuten zu einem höhermolekularen Produkt aufkondensiert. Die relative Lösungsviskosität beträgt 1,195. Die Brüden wurden kondensiert.

Vom Schmelzestrom, der in einen weiteren Korbreaktor geleitet wurde, wurde mittels einer Zahnradpumpe ein Teilstrom von 1.86 kg/h Schmelze abgezweigt, mit 2.3 g/h einer 5 Gew.-%-igen wässrigen Phosphorsäure versetzt, über einen statischen Mischer mit einem Länge-zu-Durchmesser-Verhältnis von 20 gerührt und wieder in den Hauptschmelzestrom zurückgeleitet. Direkt nach dem Zusammentreffen wurde die Phosphorsäure im gesamten Schmelzestrom mittels eines weiteren statischen Mischers homogen verteilt.

Die so behandelte Schmelze wurde in einem weiteren Korbreaktor bei 284°C, 0,7 mbar und bei einer mittleren Verweilzeit von 130 Minuten weiter den Prozessbedingungen ausgesetzt, ausgetragen und granuliert.

Die Brüden wurden in der Vakuumanlage und dahinter kondensiert.

Das erhaltene Polycarbonat hatte folgende Kennzahlen: relative Lösungsviskosität 1,201 / phenolische OH 255 [ppm] / DPC 71 [ppm] / BPA 6 [ppm] / Phenol 56 [ppm]. Das abdestillierte Phenol wurde wieder in die Diphenylcarbonat-Herstellung rückgeführt.

In Tabellel sind die YI-Werte der Polycarbonate in Abhängigkeit von den bei der Herstellung der Polycarbonate verwendeten organischen Lösungsmittel aufgeführt.

Es ist deutlich zu erkennen, dass das erfindungsgemäß hergestellte Polycarbonat ausgezeichnete YI-Werte aufweist.

Die relativen Lösungsviskositäten der erfindungsgemäß hergestellten Polycarbonate lagen zwischen 20 und 32, waren also normgerecht.

### Beispiel 2 (Stand der Technik)

### a) Herstellung und Aufarbeitung von Diphenylcarbonat (DPC)

102 g/h gasförmiges Phosgen wurden kontinuierlich in 847 g/h auf -10°C gekühltes reines Methylenchlorid gelöst. Diese Phosgenlösung wurde mit 751,45 g/h 25 Gew.-%ige Phenolatlösung, enthaltend 188,25 g/h Phenol, 593 g/h vollentsalztes Wasser und 81,2 g/h 98,5 Gew.-%ig reine NaOH-Pellets in einem inline Filter-/Dispersions-Element gemischt. Die erhaltene Reaktionsmischung wurde dann durch einen Kühler geleitet, bei einer Temperatur des Kühlmediums von -10 °C, und dann in eine erste Mischpumpe geleitet, wobei die Verweilzeit 15 Sekunden betrug. Die Reaktionslösung wurde dann in eine zweite gleichartige Mischpumpe geleitet, in der 14,94 g/h 32,15 Gew.-%ige alkalische Lösung aus 1,132 g/h N-Ethylpiperidin gelöst in 10,2 g/h Methylenchlorid zugegeben wurden, um einen pH-Wert von 9,5 bis 10 in der wässrigen Lösung im nachfolgenden gerührten Tankreaktor aufrecht zu erhalten. Die erhaltene Lösung wurde in den besagten gerührten Tankreaktor mit einer Verweilzeit von 10 Minuten und dann durch eine Zahnradpumpe in einen Schwerkraftabscheider geleitet, um die wässrige Phase zu entfernen. Die erhaltene organische DPC Lösung enthielt 214 g/h DPC und wurde einmal mit 10 Gew.-%iger Salzsäure mit einem pH-Wert von 3 ausgewaschen, um Katalysator aus der organischen Phase zu entfernen, sowie einmal mit vollentsalztem Wasser, um verbliebene Salze zu entfernen. Nach Verdampfen des Lösungsmittels wurde 99,9 Gew.-%ig reines DPC erhalten.

### b) Herstellung von Polycarbonat

In einen inerten Dreihalskolben mit einem den Kolbenboden abschabenden Rührer wurden 78,72 g DPC und 79,91 g BPA zusammen mit 120 µl 5 Gew.-%iger TPPP (Tetraphenylphosphoniumphenolat) -Lösung in Phenol gegeben. Der Druck im Kolben wurde auf 410 mbar erniedrigt und das Gemisch auf 205 °C erhitzt, um das Gemisch aufzuschmelzen und zu mischen. Der Rührer wurde auf 400 U/min eingestellt. Nach 45 Minuten wurde der Druck auf 200 mbar erniedrigt und die Temperatur auf 230 °C erhöht, nach weiteren 20 Minuten wurde der Druck auf 100 mbar erniedrigt und die Temperatur auf 245 °C erniedrigt, nach weiteren 20 Minuten wurde der Druck auf 25 mbar erniedrigt und die Temperatur auf 285 °C erhöht und dieser Druck und diese Temperatur dann 20 Minuten gehalten. Für die abschließende Polykondensation wurde die Temperatur auf 315 °C erhöht, der Druck auf 4 mbar erniedrigt und nach 5 Minuten die Rührergeschwindigkeit auf 250 U/min und dann diese Bedingungen für 20 Minuten gehalten. Anschließend wurde die Rührergeschwindigkeit auf 100 U/min verringert und dann für 20 Minuten gehalten, bevor dann die Temperatur auf 330 °C erhöht und die Unterdruckkontrolle maximal geöffnet wurde, wodurch ein Druck von kleiner 1 mbar eingestellt wurde. Nach 45 Minuten unter diesen Bedingungen wird die Rührgeschwindigkeit auf 50 U/min eingestellt, wobei nach weiteren 75 Minuten das Polycarbonat die gewünschte relative Lösungsviskosität erreicht hat.

### Beispiel 3 (Erfindungsgemäß)

Beispiel 3 wurde in der gleichen Weise wie Beispiel 2 ausgeführt, mit dem Unterschied, dass während der Herstellung des DPC anstelle von reinem Methylenchlorid als Lösungsmittel für Phosgen und N-Ethylpiperidin ein organisches Lösungsmittelgemisch enthaltend 90 Gew.-% Methylenchlorid, 4 Gew.-% Tetrachlormethan und 6 Gew.-% Chlorethan.

Wie Tabelle 2 entnommen werden kann, ergab eine visuelle Prüfung von Proben von nach Beispiel 2 und Beispiel 3 hergestelltem Polycarbonat keinerlei Unterschiede in der Qualität, beispielsweise bezüglich der Gelbstichigkeit.

Darüber hinaus sind die relativen Lösungsviskositäten von Proben von nach Beispiel 2 und Beispiel 3 hergestelltem Polycarbonat im Rahmen von statistischen Abweichungen identisch.

**Tabelle 1**

| **Lösungsmittelzusammensetzung** | **1** | **2** | **3** | **4** | **5** | **6** | **7** |
|---|---|---|---|---|---|---|---|
| Methylenchlorid Gew.-%] | 99,53 | 99,45 | 99,03 | 98,77 | 98,51 | 98,49 | 98,32 |
| Chloroform [Gew.-%] | 0 | 0 | 0,0031 | 0 | 0 | 0 | 0,0094 |
| Tetrachlormethan [Gew.-%] | 0,0778 | 0,1222 | 0,1805 | 0,2492 | 0,2905 | 0,2977 | 0,2333 |
| Chlorethan [Gew.-%] | 0,3578 | 0,4174 | 0,6781 | 0,9466 | 1,1127 | 1,1764 | 1,2669 |
| Sonstige Nebenkomponenten [Gew.-%] | 0,0344 | 0,0104 | 0,1083 | 0,0342 | 0,0868 | 0,0359 | 0,1704 |
| YI | 1,61 | 1,34 | 1,36 | 1,60 | 1,65 | 1,65 | 1,58 |

**Tabelle 2**

| **Beispiel** | **2** | **3** |
|---|---|---|
| Methylenchlorid [Gew.-%] | 100 | 90 |
| Chloroform [Gew.-%] | 0 | 0 |
| Tetrachlormethan [Gew.-%] | 0 | 4 |
| Chlorethan [Gew.-%] | 0 | 6 |
| Sonstige Nebenkomponenten [Gew.-%] | 0 | 0 |
| **Visuelle Bewertung** | **gut** | **gut** |
| **Relative Lösungsviskosität** | **28,5 +/- 0,38 (*1)** | **28,75 +/- 0,65 ^{(*2)}** |

| | | |
|---|---|---|
| (*1) Mittelwert und Standardabweichung aus 3 Proben von Polycarbonat, das gemäß Beispiel 2 hergestellt wurde. (*2) Mittelwert und Standardabweichung aus 2 Proben von Polycarbonat, das gemäß Beispiel 3 hergestellt wurde. | | |

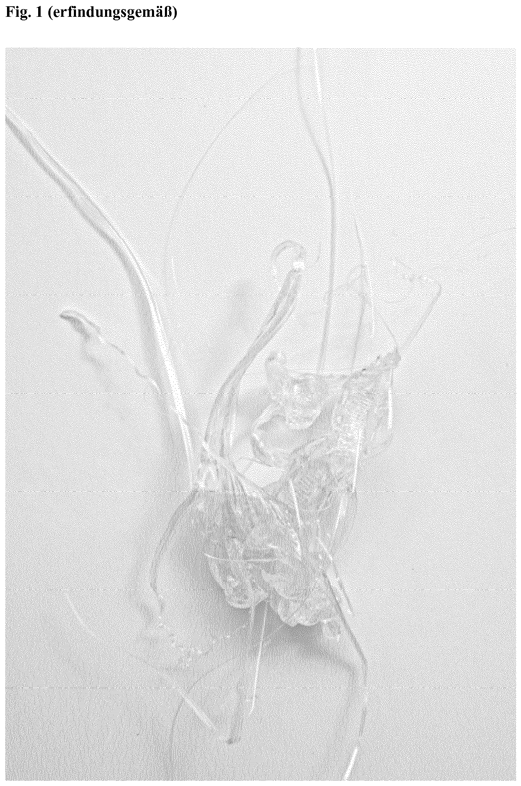

In Fig. 1 ist ein Foto einer Probe von nach Beispiel 3 (erfindungsgemäß) hergestelltem Polycarbonat wiedergeben.

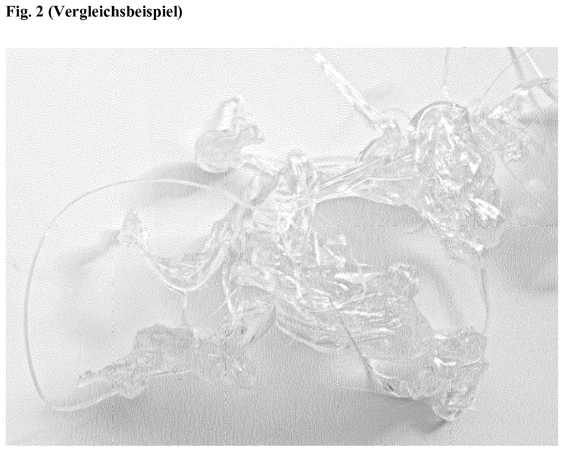

In Fig. 2 ist ein Foto einer Probe von nach Beispiel 2 (Stand der Technik) hergestelltem Polycarbonat wiedergeben.

Es sind keinerlei visuellen Unterschiede der Qualität der nach Beispiel 2 und Beispiel 3 hergestellten Polycarbonate zu erkennen.

## Patentansprüche

1. Verfahren zur Herstellung eines Polycarbonats nach dem Schmelzumesterungsverfahren, das wenigstens folgende Schritte umfasst:
(a) Lösen eines Monophenols in einer wässriger Phase und Lösen eines Carbonylhalogenids in einem organischen Lösungsmittel,
(b) Umsetzung des in der wässrigen Phase gelösten Monophenols mit dem in dem organischen Lösungsmittel gelösten Carbonylhalogenid zu einem in dem organischen Lösungsmittel gelösten Diarylcarbonat,
(c) Zuführung dieses in dem organischen Lösungsmittel gelösten Diarylcarbonats zu Schritt (d),
(d) Abtrennung des organischen Lösungsmittel von dem in Schritt (b) erhaltenen Diarylcarbonat und Zuführung dieses Diarylcarbonats zu Schritt (e) und Rückführung des organischen Lösungsmittels zu Schritt (a),
(e) Umsetzung eines oder mehrerer Diphenole mit dem Diarylcarbonat aus Schritt (d) zu einem Polycarbonat und einem Monophenol,
wobei das organische Lösungsmittel folgende Komponenten enthält:
| | |
|---|---|
| Methylenchlorid | 0 bis 99,65 Gew.-%, |
| Chlorbenzol | 0 bis 99,65 Gew-% |
| Chloroform | 0 bis 99,65 Gew-% |
| Chlorethan | 0,3 bis 10 Gew.-%, |
| Tetrachlormethan | 0,05 bis 7,0 Gew.-%, |
| sonstige Komponenten | 0 bis 2,0 Gew.-%, |
wobei die Summe der Gehalte von Methylenchlorid, Chlorbenzol und Chloroform mindestens 81,00 Gew.-% und höchstens 99,65 Gew.-% beträgt,
und wobei Summe der Gehalte von Methylenchlorid, Chlorbenzol und Chloroform vermehrt um die Summe der Gehalte von Chlorethan, Tetrachlorethan und der sonstigen Komponenten 100 Gew.-% beträgt.

2. Verfahren nach Anspruch 1, wobei der Gehalt von Chlorethan im organischen Lösungsmittel von 0,9 Gew.-% bis 8,0 Gew.-%, bevorzugt von 2,0 Gew-% bis 7,0 Gew.-%, besonders bevorzugt von 2,5 Gew.-% bis 6,0 Gew.-% beträgt.

3. Verfahren nach Anspruch 1 oder 2, wobei der Gehalt von Tetrachlormethan im organischen Lösungsmittel von 0,2 Gew.-% bis 7,0 Gew.-%, bevorzugt von 0,5 bis 6,0 Gew.-%, besonders bevorzugt von 1,0 Gew.-% bis 4,0 Gew.-% beträgt.

4. Verfahren nach Anspruch 2 oder 3, wobei im organischen Lösungsmittel der Gehalt von Chlorethan von 0,9 Gew.-% bis 8,0 Gew.-% und der Gehalt von Tetrachlormethan von 0,2 Gew.-% bis 7,0 Gew.-% beträgt, bevorzugt der Gehalt von Chlorethan von 2,0 Gew-% bis 7,0 Gew.-% und der von Tetrachlormethan von 0,5 bis 6,0 Gew.-% beträgt, besonders bevorzugt der Gehalt von Chlorethan von 2,5 Gew.-% bis 6,0 Gew.-% und der von Tetrachlormethan von 1,0 Gew.-% bis 4,0 Gew.-% beträgt.

5. Verfahren nach einem der vorhergehenden Ansprüche, wobei in Schritt (d) das organische Lösungsmittel durch Destillation abgetrennt wird.

6. Verfahren nach einem der vorhergehenden Ansprüche, wobei das in Schritt (e) erhaltene Monophenol wieder in Schritt (a) eingesetzt wird.

7. Verfahren nach einem der vorhergehenden Ansprüche, wobei das organische Lösungsmittel nach der Abtrennung in Schritt (d) ohne weitere Abtrennung von flüssigen oder gasförmigen Bestandteilen wieder Schritt (a) zugeführt wird.

8. Verfahren nach einem der vorhergehenden Ansprüche, wobei das organische Lösungsmittel in einem in Bezug auf dieses Lösungsmittel geschlossenen Kreislauf geführt wird,
und wobei die Menge des organischen Lösungsmittels während der Durchführung des Verfahrens nicht vermehrt wird,
und wobei erst nach einer zeitlichen Periode, in der pro Massenanteil des organischen Lösungsmittels 1000 bis 15.000, bevorzugt 1.500 bis 12.000, besonders bevorzugt 1.750 bis 8.000, ganz besonders bevorzugt 2.000 bis 6.000 Massenanteile des Diarylcarbonats gemäß den Schritten (a) bis (d) hergestellt werden, 100 % oder weniger, bevorzugt 70 % oder weniger, besonders bevorzugt 50 % oder weniger, ganz besonders bevorzugt 30 % oder weniger des organischen Lösungsmittels ersetzt oder aufgereinigt werden.

9. Verfahren nach einem der vorhergehenden Ansprüche, wobei das organische Lösungsmittel in einem in Bezug auf dieses Lösungsmittel geschlossenen Kreislauf geführt wird,
und wobei die Menge des organischen Lösungsmittels während der Durchführung des Verfahrens nicht vermehrt wird,
und wobei innerhalb einer zeitlichen Periode, in der pro Massenanteil des organischen Lösungsmittels 1000 bis 15.000, bevorzugt 1.500 bis 12.000, besonders bevorzugt 1.750 bis 8.000, ganz besonders bevorzugt 2.000 bis 6.000 Massenanteile des Diarylcarbonats gemäß den Schritten (a) bis (d) hergestellt werden, maximal ein Anteil von 20 %, bevorzugt maximal ein Anteil von 10 %, besonders bevorzugt maximal ein Anteil von 5 %, ganz besonders bevorzugt maximal ein Anteil von 2 %, insbesondere maximal ein Anteil von 1 Gew.-%, des organischen Lösungsmittels dem Lösungsmittel entnommen wird und gleichzeitig durch eine dem entnommenen Anteil an organischem Lösemittelgemisch entsprechende Menge eines Lösungsmittelgemischs umfassend ein oder mehrere Komponenten ausgewählt aus Methylenchlorid, Chlorbenzol und Chloroform ergänzt wird.

10. Polycarbonat, erhältlich nach einem Verfahren nach einem der vorhergehenden Ansprüche.

11. Polycarbonat nach Anspruch 10, wobei es einen YI-Wert von kleiner oder gleich 2, bevorzugt von kleiner oder gleich 1,7 aufweist.

12. Polycarbonat nach Anspruch 10 oder Anspruch 11, wobei es eine relative Lösungsviskosität von 15 bis 40, bevorzugt von 20 bis 35 aufweist.

13. Organisches Lösungsmittel, geeignet zur Herstellung von Polycarbonaten nach einem der Ansprüche 1 bis 15, wobei das organische Lösungsmittel folgende Komponenten aufweist:
| | |
|---|---|
| Methylenchlorid | 0 bis 99,65 Gew.-%, |
| Chlorbenzol | 0 bis 99,65 Gew-% |
| Chloroform | 0 bis 99,65 Gew-% |
| Chlorethan | 0,3 bis 10 Gew.-%, |
| Tetrachlormethan | 0,05 bis 7,0 Gew.-%, |
| sonstige Komponenten | 0 bis 2,0 Gew.-%, |
und wobei die Summe der Gehalte von Methylenchlorid, Chlorbenzol und Chloroform mindestens 81,00 Gew.-% und höchstens 99,65 Gew.-% beträgt, und wobei Summe der Gehalte von Methylenchlorid, Chlorbenzol und Chloroform vermehrt um die Summe der Gehalte von Chlorethan, Tetrachlorethan und der sonstigen Komponenten 100 Gew.-% beträgt.
